Europäisches Patentamt

European Patent Office

Office européen des brevets

(11) Publication number: 0 036 260
B2

# NEW EUROPEAN PATENT SPECIFICATION

(45) Date of publication of the new patent specification: 14.12.88

(51) Int. Cl.⁴: **C 10 L 1/02**

(21) Application number: **81300864.6**

(22) Date of filing: **03.03.81**

(54) Preparation of a motor spirit blending component.

(30) Priority: **07.03.80 GB 8007845**

(43) Date of publication of application:
**23.09.81 Bulletin 81/38**

(45) Publication of the grant of the patent:
**30.05.84 Bulletin 84/22**

(45) Mention of the opposition decision:
**14.12.88 Bulletin 88/50**

(84) Designated Contracting States:
**BE DE FR GB IT NL**

(56) References cited:
**GB-A- 545 464
GB-A-1 249 016
US-A-2 480 940
US-A-3 902 870
US-A-3 912 463
US-A-3 966 586
Alkyl Ethers as Motor Fuels, A.F. Talbot,
Proceedings Refining Department, Vol.58, API
1979, pp.205-218**

(73) Proprietor: **The British Petroleum Company
p.l.c.
Britannic House Moor Lane
London EC2Y 9BU (GB)**

(72) Inventor: **Haelsig, Claus-Peter**
**Deutsche BP Aktiengesellschaft Postfach 60 03
40**
**D-2000 Hamburg 60 (DE)**
Inventor: **Hoenig, Helmar**
**Deutsche BP Aktiengesellschaft Postfach 60 03
40**
**D-2000 Hamburg 60 (DE)**
Inventor: **Petzny, Wilfried**
**Deutsche BP Aktiengesellschaft Postfach 60 03
40**
**D-2000 Hamburg 60 (DE)**
Inventor: **Raymond, David William**
**The British Petroleum Company Ltd. Britannic
House**
**Moor Lane London EC2Y 9BU (GB)**

(74) Representative: **MacLeod, Malcolm et al
c/o The British Petroleum Company plc Patents
Division Chertsey Road
Sunbury-on-Thames Middlesex, TW16 7LN (GB)**

EP 0 036 260 B2

Courier Press, Leamington Spa, England.

## EP 0 036 260 B2

**Description**

This invention relates to a process for the preparation of a motor spirit blending component.

Motor spirit is a blend of several components which can be selected, inter alia, from straight run distillate, catalytic reformate, isomerate, alkylate, catalytically cracked gasoline and steam cracked gasoline.

Straight run distillates alone are inadequate both in quantity and quality for modern requirements and for many years it has been common practice to increase the yield of motor spirit component from crude oil by catalytically cracking heavier fractions.

The catalytic cracking of heavier petroleum fractions normally boiling in the range 350°C to 550°C, to lighter products, particularly to gasoline, conventionally uses a fluidised bed of catalyst in a riser, or riser and reactor, and a circulation system through which spent catalyst passes to a regenerator containing a fluidised bed of catalyst and back to the riser. Catalytic cracking produces a spectrum of products ranging from gases through light cat cracked gasoline and heavy cat cracked gasoline to decanted oil.

These products are initially separated in a main fractionator and components with boiling points up to a value in the range 150°C to 300°C are taken in an overhead stream. This stream is then fed to an absorber stripper or similar system where most of the ethane and lighter components are removed as an overhead stream. The bottom stream containing propylene and higher boiling components is fed to a debutaniser where most of the butane and lighter components are taken overhead. The debutaniser bottom stream is either taken as a motor spirit component or is fed to a splitter to produce separate light and heavy gasoline and frequently also a gas oil stream as a bottom product. The debutaniser top stream is fed to a depropaniser where it is split into a $C_3$ top stream and a $C_4$ bottom stream.

The $C_4$ and lighter products are frequently separated into a $C_4$ stream, a $C_3$ stream and a light gas stream. The $C_4$ stream contains significant quantities of isobutane, n-butane, isobutene and normal butenes and is useful as a source of alkylation feedstock for the production of motor spirit alkylate.

One proposal to utilise the $C_4$ stream involves etherification of the isobutene contained therein with a lower alcohol, e.g. methanol to produce methyl tertiary butyl ether (MTBE). MTBE, because of its high octane rating, good volatility characteristics and lack of chemical activity is a very desirable motor spirit component, particularly for blending with catalytic reformate which has a deficiency of high octane components in its lower boiling end (front end).

The steam cracking of naphtha for the production of olefins is also a process of major importance. As with cat cracking, a spectrum of products is produced including ethylene, propylene, a $C_4$ fraction and material boiling in the gasoline range. The latter after treatment to reduce its unsaturation which would otherwise lead to problems with gum formation is a suitable blending component for motor spirit.

A complex product recovery system is necessary including a demethaniser for splitting between $C_1$ and higher products, a de-ethaniser for splitting between $C_2$ and higher products, a depropaniser for splitting between $C_3$ and higher products and a debutaniser for splitting between $C_4$ and raw gasoline. Reference is made to Modern Petroleum Technology, 4th Edition, edited by Hobson and Pohl and published by Applied Science Publishers Limited, Barking, Essex in 1973 on behalf of the Institute of Petroleum, at pages 449—453, for details of a steam cracking process and its product recovery system.

The $C_4$ stream is generally solvent extracted for butadiene recovery. After the latter has been removed the remaining $C_4$ contains a higher proportion of isobutene than a cat cracked $C_4$ stream.

Process have been disclosed in the Oil and Gas Journal, April 9th 1979, pages 149—152, and Hydrocarbon Processing, December 1979, pages 109—113, for the reaction of $C_4$ streams with methanol to produce MTBE. In each case, however, the reaction product is fractionated to obtain MTBE as a relatively pure single compound, excess or unreacted methanol is recovered and recycled and unreacted material containing isobutene, isobutane and linear butenes and butanes is used for other purposes, e.g. alkylation or polymerisation. The MTBE is added to a motor spirit blend as a single compound.

The Oil and Gas Journal, ibid, also discloses an analogous process for the production of methyl tertiary amyl ether.

GB—A 1 249 016 (Reference 1) discloses the reaction of material obtained from cracking of hydrocarbons with a lower alcohol e.g. methanol. However the material which is subjected to the reaction with the alcohol has a narrow boiling fraction boiling in the range 15° to 170°F (−10 to 77°C). This is obtained by feeding the effluent from the cracking reactor to a distillation column in which the effluent is separated into a plurality of fractions.

GB 1 249 016 points out (page 5, lines 39—46) that low molecular weight hydrocarbons contribute disproportionately to the total volatility of the blend. It would be possible to remove unreacted $C_4$ hydrocarbons by the use of a debutaniser.

US 3 092 870 uses a cracked gasoline boiling in the range $C_5$ upwards. The feedstock is thus a material from which $C_4$ and lower boiling materials have been removed for example by the conventional debutaniser.

US 3 912 463 discloses the etherification of tertiary olefins with alcohols to give ethers. Although the specification refers to using hydrocarbons having between 3 and 7 carbon atoms in the molecule, the only specific mixture disclosed is a mixture of $C_4/C_5$ hydrocarbons and a preference is expressed for the use of a $C_4$ hydrocarbon stream. Such a narrow $C_4$ cut is stated to contribute to the efficiency of the etherification

2

process when compared to wider cuts such as $C_3$ to $C_6$ hydrocarbon fractions (column 3, lines 29—45).

Processes described in the prior art for the production of MTBE for use as a motor gasoline component, or for the etherification of a narrow cut containing isobutene together with higher iso-olefins, have suffered from the disadvantage that substantial amounts of energy are required before and after the etherification step (a) to separate the narrow cut for the etherification reaction and (b) to fractionate the product.

We have discovered that an etherification stage can be inserted into the recovery section of a cat or steam cracker at the point, typically downstream of an absorber/stripper, where $C_2$ hydrocarbons and lower boiling materials have preferably been removed, leaving a mixture consisting substantially of $C_3$, $C_4$ and higher boiling hydrocarbons up to a final boiling point within the range of 150 to 300°C.

This enables the pre-reaction fractionation step to be omitted and reduces $C_4$ removal requirements in the product fractionator (debutaniser) with consequent saving in capital and operating costs.

Thus according to the present invention, there is provided a process for the production of a motor spirit blending component from

(a) a hydrocarbon feedstock containing isobutene and reactive tertiary olefins of higher carbon number and having a final boiling point of at least 150°C, by a process including,

(b) a step of removing $C_4$ hydrocarbons characterised in that the feedstock (a) before it is fed to the step of removing $C_4$ hydrocarbons (b) is:

(c) reacted with a lower aliphatic alcohol to produce a product containing tertiary ethers,

(i)   in the presence of an etherification catalyst,

(ii)   at a temperature in the range 30—200°C,

(iii)   a pressure in the range atmospheric to 100 bar,

(iv)   a liquid hourly space velocity in the range 0.1 to 20 vol/vol/h, and

(v)   a molar ratio of alcohol to reactive $C_4$—$C_7$ tertiary olefins in the range 0:1 to 10:1.

The molar ratio of alcohol to olefins is defined with respect to $C_4$—$C_7$ olefins for analytical reasons, although high tertiary olefins also react and the products will contain ethers derived from these materials.

The process is particularly applicable to that fraction from a catalytic cracker product recovery system which would normally be fed to the debutaniser, i.e., $C_3$ and higher hydrocarbons.

The process is also applicable to that fraction from a steam cracker product recovery system which would normally be fed to the depropaniser, i.e., $C_3$ and higher hydrocarbons.

Preferably the feedstock has a final boiling point not greater than 300°C.

By lower alcohol is meant an alcohol containing from 1 to 6 carbon atoms per molecule. The preferred alcohol is methanol.

The catalysts are preferably relatively high molecular weight solid carbonaceous materials containing at least one —$SO_3H$ group per molecule as the functional group.

The most preferred are strongly acidic cation exchange resins consisting essentially of a sulphonated polystyrene resin, for instance a divinylbenzene cross-linked polystyrene matrix having 0.5 to 20% and preferably 4 to 16% of copolymerised divinylbenzene attached to which are ionisable or functional nuclear sulphonic acid groups. These resins are manufactured and sold commercially under various trade names.

The temperature at which the etherification reaction is carried out is preferably in the range 50° to 80°C.

The pressure should be sufficient to maintain the reactants in the liquid phase. Pressures between 10 and 20 bar are preferred.

Because of azeotrope formation between the alcohols, particularly methanol, and butenes and pentenes, it is desirable for the reaction product to have a relatively low alcohol content prior to fractionation.

This is particularly so when methanol is employed as the alcohol and methanol is to be removed from the reaction products after fractionation. In certain circumstances methanol is an undesirable product in gasoline fractions because of the sensitivity of the Reid Vapour Pressure to relatively low concentrations of methanol.

If, however, methanol is intended to form a significant component of the final blend of motor spirit, then the above consideration does not apply.

It is therefore advisable from the former point of view to operate with a low alcohol content in the feedstock and therefore with a low molar ratio of alcohol to reactive tertiary olefins.,

The quantity of alcohol supplied may be less than the stoichiometric equivalent required for complete etherification of the reactive tertiary olefins.

If this is the case then an alkyl tertiary butyl ether is formed preferentially because isobutene reacts more readily than higher boiling tertiary olefins. When progressively large quantities of alcohol are added, progressively more of the reactive higher tertiary olefins are etherified but the conversion of alcohol decreases leaving a higher proportion of unreacted alcohol in the reaction product.

Preferably the molar ratio of alcohol to reactive tertiary $C_4$—$C_7$ olefin is in the range 0.3:1 to 1.5:1.

Alcohol addition is most limited when the objective is to achieve the optimum gain in octane number and the optimum increase in the production of gasoline for the least use of alcohol; larger additions of alcohol are used when it is desired to etherify more of the olefins.

Increasing the ether content and reducing the olefin content results in a gasoline of reduced photochemical activity, reduced vapour pressure, reduced bromine number, increased octane number and increased density.

# EP 0 036 260 B2

Insofar as the liquid hourly space velocity (LHSV) of the feedstock is concerned, a compromise must be reached since decreasing the space velocity results in high conversions and less unreacted alcohol whereas increasing the space velocity has the opposite effect. The preferred range lies between 0.5 and 5 vol/vol/hour.

The MTBE and other ethers formed under the above conditions are retained in the gasoline produced by the later fractionation stages of a recovery section. The non-etherified $C_4$ hydrocarbons, from which the isobutene has been substantially removed by etherification, may be recovered in the normal way by distillation without an additional step being necessary.

Unreacted methanol, ethanol or propanol can be removed by washing the reaction product with water. Washing can be carried out before or after the fractionation of the product. However, because of azeotrope formation it is preferred to carry out this operation before.

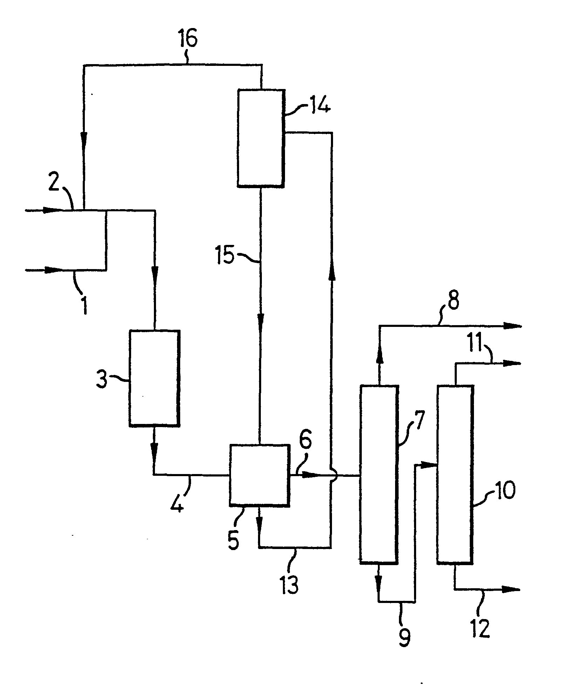

The invention is illustrated with reference to the accompanying drawing which is a simplified flow diagram.

A stream 1 containing debutaniser feedstock from a fluidised cat cracking unit (not shown) is joined by a line 2 containing methanol and the combined stream is fed to an etherification reactor 3 containing a cation exchange resin in the hydrogen form. Line 4 takes the product from the reactor 3 to a washer 5 wherein it is scrubbed with water to remove unreacted methanol. The methanol-free product is then passed by line 6 to a debutaniser 7 which splits it into top and bottom products. The former is removed as LPG by line 8 after a conventional purification treatment (not shown).

The bottom product which boils in the gasoline boiling range is passed by line 9 to a distillation column 10 which splits it into light and heavy cat cracked spirits. After conventional purification treatments, again not shown, these are removed by lines 11 and 12 respectively.

Wash water containing recovered methanol is taken from washer 5 by line 13 and passed to a column 14 in which the water and methanol are separated by distillation. Water is returned to the washer by line 15 and methanol is recycled by line 16 to join the methanol feed line 2.

The invention is further illustrated by the following Examples 1—7.

## Examples

The feedstock was a simulated cat cracker debutaniser feed prepared by adding 12% by weight of a $C_4$ cut to 88% by weight cat cracked stabiliser feed boiling in the range 29° to 202°C and derived from Forties vacuum distillate.

The simulated debutaniser feedstock boiled in the range −7°C to 202°C and had the following analysis.

| Material | Concentration % wt | |
|---|---|---|
| Isobutene | 2.1 | |
| Reactive tertiary isopentenes | 3.7 | |
| Reactive tertiary isohexenes | 2.9 | 1.6 mol/Kg |
| Reactive tertiary isoheptenes | 3.4 | |
| Relatively inert hydrocarbons | 87.9 | |

In each example, the catalyst was a sulphonated divinyl benzene cross-linked polystyrene cation exchange resin sold under the trade name Lewatit SPC 118 by Farbenfabriken Bayer AG. Methanol was the etherifying agent, the maximum reaction temperature was 74°C and the pressure was 15 bar. Products were analysed by gas chromatography. Octane number measurements were made after the products had been washed with water to remove untreated methanol.

## Examples 1, 2 and 3

In Examples 1—3, methanol was supplied so that the molar ratio of methanol to reactive $C_4$—$C_7$ tertiary olefins was 0.72:1 and the LHSV was varied.

The following results were obtained.

4

| Example | LHSV | Isobutene conversion % | Selectivity to MTBE % | Isopentene conversion % | Selectivity to MTAE % |
|---------|------|------------------------|----------------------|--------------------------|-----------------------|
| 1 | 1 | 91 | 94 | 51 | 100 |
| 2 | 2 | 91 | 94 | 44 | 100 |
| 3 | 3 | 90 | 95 | 36 | 100 |

| Example | LHSV | Isohexene conversion % | Selectivity to MTHEXE % | Isoheptene conversion % | Selectivity to MTHEPE % | Methanol conversion % |
|---------|------|------------------------|--------------------------|--------------------------|--------------------------|------------------------|
| 1 | 1 | 30 | 100 | 17 | 100 | 74 |
| 2 | 2 | 21 | 100 | 14 | 100 | 67 |
| 3 | 3 | 14 | 100 | 12 | 100 | 61 |

MTBE=methyl tert-butyl ether
MTAE=methyl tert-amyl ether
MTHEXE=methyl tert-hexyl ether
MTHEPE=methyl tert-heptyl ether

Octane numbers were determined for Examples 1 to 3 on the feedstock, the products and the products with different quantities of TEL added.

The results set out in the following Table I show the improvement of octane number obtained as a function of the LHSV.

TABLE I

|  |  | Original feed | Example 1 | Example 2 | Example 3 |
|--|--|---------------|-----------|-----------|-----------|
| RON | +0.4 g TEL/l as Pb | 96.8 | +0.4 | +0.4 | +0.4 |
| MON |  | 85.2 | +0.4 | +0.4 | +0.4 |
| RON | 0.15 g TEL/l as Pb | 95.1 | +0.3 | +0.3 | +0.3 |
| MON |  | 83.7 | +0.5 | +0.5 | +0.5 |
| RON | Clear | 91.5 | +0.3 | +0.3 | +0.3 |
| MON |  | 80.6 | +0.4 | +0.4 | +0.4 |

Examples 4, 5 and 6

In Examples 4 to 6, an LHSV of 1 was used throughout and the molar ratio of methanol to reactive $C_4$—$C_7$ tertiary olefin was varied.

The following results were obtained, Example 1 being again included because it fits into the pattern.

| Example | Molar ratio of methanol to olefins | Isobutene conversion % | Selectivity to MTBE % | Isopentene conversion % | Selectivity to MTAE % |
|---------|-------------------------------------|------------------------|----------------------|--------------------------|-----------------------|
| 4 | 0.36:1 | 80 | 99 | 26 | 100 |
| 5 | 0.53:1 | 89 | 94 | 36 | 100 |
| 1 | 0.72:1 | 91 | 94 | 51 | 100 |
| 6 | 0.97:1 | 92 | 94 | 60 | 100 |

5

| Example | Molar ratio of methanol to olefins | Isohexene conversion % | Selectivity to MTHEXE % | Isoheptene conversion % | Selectivity to MTHEPE % | Methanol conversion % |
|---|---|---|---|---|---|---|
| 4 | 0.36:1 | 12 | 100 | 8 | 100 | 98 |
| 5 | 0.53:1 | 30 | 100 | 17 | 100 | 74 |
| 1 | 0.72:1 | 30 | 100 | 17 | 100 | 74 |
| 6 | 0.97:1 | 35 | 100 | 18 | 100 | 58 |

Octane numbers were determined for Examples 4 to 6 on the feedstock, the products and the products with different quantities of TEL added.

The results set out in the following Table II show the improvement in octane number obtained as a function of the mole ratio of methanol to reactive $C_4$—$C_7$ tertiary olefins.

TABLE II

| | Original feed | Example 4 | Example 5 | Example 1 | Example 6 |
|---|---|---|---|---|---|
| RON +0.4 g TEL/l as Pb | 96.8 | +0.5 | +0.4 | +0.4 | +0.5 |
| MON | 85.2 | +0.3 | +0.3 | +0.4 | +0.6 |
| RON +0.15 g TEL/l as Pb | 95.1 | +0.1 | +0.1 | +0.3 | +0.4 |
| MON | 83.7 | +0.4 | +0.4 | +0.4 | +0.8 |
| RON Clear | 91.5 | +0.3 | +0.3 | +0.3 | +0.5 |
| MON | 80.6 | +0.4 | +0.4 | +0.4 | +0.7 |

Example 7

A similar feedstock to that used in the previous Examples but containing twice as much isobutene and a total of 1.9 mole/Kg of reactive tertiary $C_4$—$C_7$ olefins was etherified with methanol.

Etherification conditions were as follows:

```
Catalyst      ⎫
Temperature   ⎬   as before
Pressure      ⎭
LHSV              1
Molar ratio of methanol
   to reactive tertiary C4—C7
   olefins       1.52:1
```

Octane numbers were determined for the feedstock and the product, and for the feedstock and the product with different quantities of TEL added.

The following results were obtained.

| Substance | Increase in octane No. | |
|---|---|---|
| | MON | RON |
| Product without TEL | 1.6 | 1.3 |
| Product+0.15 g/l TEL as Pb | 1.4 | 0.8 |
| Product+0.4 g/l TEL as Pb | 1.5 | 0.8 |

TEL=Tetra ethyl lead
MON=Motor Octane No.
RON=Research Octane No.

Examples 8 to 11

A similar feedstock to that used in the previous Examples but containing 3.15% wt isobutene, 4.7% wt isopentenes, 3.1% wt isohexenes and 3.4% wt isoheptenes and a total of 1.9 mole/Kg of reactive $C_4$—$C_7$ tertiary olefins was etherified with methanol.

The catalyst and pressure were as before. The molar ratio of methanol to reactive tertiary $C_4$—$C_7$ olefines was 0.93 and the LHSV was 1.

The following results were obtained.

| Example | Temperature °C | Isobutene conversion % | Selectivity to MTBE % | Isopentene conversion % | Selectivity to MTAE % |
|---|---|---|---|---|---|
| 8 | 74 | 93 | 98 | 61 | 100 |
| 9 | 70 | 93 | 100 | 56 | 100 |
| 10 | 66 | 93 | 100 | 51 | 100 |
| 11 | 62 | 92 | 100 | 46 | 100 |

| Example | Temperature °C | Isohexene conversion % | Selectivity to MTHEXE % | Isoheptene conversion % | Selectivity to MTHEPE % | Methanol conversion % |
|---|---|---|---|---|---|---|
| 8 | 74 | 35 | 100 | 20 | 100 | 64 |
| 9 | 70 | 29 | 100 | 17 | 100 | 62 |
| 10 | 66 | 24 | 100 | 15 | 100 | 60 |
| 11 | 62 | 20 | 100 | 15 | 100 | 54 |

Octane numbers were determined for Examples 8—10 on the feedstock, the products, and the products with TEL added.

The results set out in the following Table III show the improvement in octane number obtained as a function of the temperature.

TABLE III

| | | Original feed | Example 8 | Example 9 | Example 10 |
|---|---|---|---|---|---|
| RON | +0.15 g TEL/l as PB | 96.5 | +0.7 | +0.6 | +0.6 |
| MON | | 85.0 | +1.1 | +1.1 | +1.1 |
| RON | Clear | 92.9 | +0.5 | +0.6 | +0.6 |
| MON | | 81.8 | +0.8 | +1.0 | +1.0 |

**Claims**

1. A process for the production of a motor spirit blending component containing tertiary alkyl ethers from a hydrocarbon feedstock containing isobutene and other tertiary olefins by reacting the hydrocarbon feedstock with a lower aliphatic alcohol in the presence of an etherification catalyst at a temperature of 30—200°C and a pressure in the range atmospheric to 100 bar is characterised in that
(1) the hydrocarbon feedstock comprises isobutene and reactive tertiary olefins of higher carbon number and has a final boiling point of at least 150°C which hydrocarbon feedstock is derived from a catalytic or steam cracker,
(2) the etherification is carried out at a liquid hourly space velocity of 0.1 to 20 vol/vol/h and with a molar ratio of alcohol to reactive $C_4$—$C_7$ tertiary olefins in the range 0.1:1 to 10:1, and
(3) the cracker product is fed to a debutaniser for the first time after the etherification step.

2. A process according to claim 1 wherein the feedstock is debutaniser feedstock from a catalytic cracker.

3. A process according to claim 1 wherein the feedstock is depropaniser feedstock from a steam cracker.

4. A process according to any of the preceding claims wherein the feedstock has a final boiling point not greater than 300°C.

5. A process according to any of the preceding claims wherein the alcohol is methanol.

7

6. A process according to any of the preceding claims wherein the catalyst is a sulphonated cation exchange resin in the hydrogen form.

7. A process according to any of the preceding claims wherein the etherification reaction is carried out at a temperature in the range 50° to 80°C.

8. A process according to any of the preceding claims wherein the etherification reaction is carried out under a pressure in the range 10 to 20 bar.

9. A process according to any of the preceding claims wherein the molar ratio of alcohol to reactive tertiary olefin is in the range 0.3:1 to 1.5:1.

10. A process according to any of the preceding claims wherein the liquid hourly space velocity of the feedstock lies in the range 0.5 to 5 vol/vol/hour.

## Patentansprüche

1. Verfahren zur Herstellung einer Komponente für ein Motorbrennstoffgemisch, die tertiäre Alkylether enthält, aus einem Isobuten und andere tertiäre Olefine enthaltenden Kohlenwasserstoff-Einsatzmaterial durch Reaktion des Kohlenwasserstoff-Einsatzmaterials mit einem niederen aliphatischen Alkohol in Gegenwart eines Veretherungs-Katalysators bei einer Temperatur von 30°C bis 200°C und einem Druck im Bereich von Atmosphärendruck bis 100 bar, dadurch gekennzeichnet, daß

(1) das Kohlenwasserstoff-Einsatzmaterial Isobuten und reaktionsfähige tertiäre Olefine mit einer höheren Kohlenstoff-Zahl enthält und einen Siedeendpunkt von wenigstens 150°C besitzt, wobei das Kohlenwasserstoff-Einsatzmaterial aus einer katalytischen oder einer Dampf-Spaltanlage stammt,

(2) die Veretherung mit einer stündlichen Flüssigkeits-Raumgeschwindigkeit von 0,1 bis 20 Vol./Vol./h und einem Stoffmengenverhältnis ("Molverhältnis") des Alkohols zu den Reaktionsfähigen tertiären $C_4$- bis $C_7$-Olefinen im Bereich von 0,1:1 bis 10:1 durchgeführt wird und

(3) das Produkt der Spaltanlage nach dem Schritt der Veretherung zum ersten Mal in einen Debutanisator eingespeist wird.

2. Verfahren nach Anspruch 1, worin das Einsatzmaterial Debutanisator-Einsatzmaterial aus einer katalytischen Spaltanlage ist.

3. Verfahren nach Anspruch 1, worin das Einsatzmaterial Depropanisator-Einsatzmaterial aus einer Dampf-Spaltanlage ist.

4. Verfahren nach irgendeinem der vorhergehenden Ansprüche, worin das Einsatzmaterial einen Siedeendpunkt von nicht mehr als 300°C besitzt.

5. Verfahren nach irgendeinem der vorhergehenden Ansprüche, worin der Alkohol Methanol ist.

6. Verfahren nach irgendeinem der vorhergehenden Ansprüche, worin der Katalysator ein sulfoniertes Kationenaustauschharz in der Wasserstoff-Form ist.

7. Verfahren nach irgendeinem der vorhergehenden Ansprüche, worin die Veretherungsreaktion bei einer Temperatur im Bereich von 50°C bis 80°C durchgeführt wird.

8. Verfahren nach irgendeinem der vorhergehenden Ansprüche, worin die Veretherungsreaktion unter einem Druck im Bereich von 10 bis 20 bar durchgeführt wird.

9. Verfahren nach irgendeinem der vorhergehenden Ansprüche, worin das Stoffmengenverhältnis des Alkohols zu den reaktionsfähigen tertiären Olefinen im Bereich von 0,3:1 bis 1,5:1 liegt.

10. Verfahren nach irgendeinem der vorhergehenden Ansprüche, worin die stündliche Flüssigkeits-Raumgeschwindigkeit des Einsatzmaterials in dem Bereich von 0,5 bis 5 Vol./Vol./h liegt.

## Revendications

1. Un procédé de production d'un composant de mélange d'un combustible pour moteurs contenant des éthers alkyles tertiaires à partir d'un hydrocarbure constituant la charge d'alimentation, contenant de l'isobutène et d'autres oléfines tertiaires en faisant réagir l'hydrocarbure constituant la charge d'aliminentation avec un alcool aliphatique inférieur en présence d'un catalyseur d'éthérification à une température de 30 à 200°C et sous une pression comprise dans la gamme atmosphèrique jusqu'à 100 bars est caractérisé en ce que (1) l'hydrocarbure constituant la charge d'alimentation contient de l'isobutène et des oléfines tertiaires réactives d'indice de carbone plus élevé et a un point d'ébullition final de 150°C au moins, lequel hydrocarbure constituant la charge d'alimentation est dérivé d'une unitè de craquage catalytique ou à la vapeur,

(2) l'éthérification est effectuée à une vitesse spatiale horaire liquide comprise entre 0,1 et 20 vol/vol/h et avec un rapport molaire entre l'alcool et les oléfines tertiaires réactives $C_4$—$C_7$ dans la plage de 0,1:1 à 10:1, et

(3) le produit de l'unité de craquage est amené vers un débutaniseur pour la première fois après l'opération d'éthérification.

2. Un procédé selon la revendication 1, dans lequel la charge d'alimentation est une charge d'alimentation de débutaniseur provenant d'une unité de craquage catalytique.

3. Un procédé selon la revendication 1, dans lequel la charge d'alimentation est une charge d'alimentation de dépropaniseur provenant d'une unité de craquage à la vapeur.

4. Un procédé selon l'une quelconque des revendications précédentes, dans lequel la charge d'alimentation a un point d'ébullition final qui n'est pas supérieur à 300°C.

5. Un procédé selon l'une quelconque des revendications précédentes, dans lequel l'alcool est du méthanol.

6. Un procédé selon l'une quelconque des revendications précédentes, dans lequel le catalyseur est une résine échangeuse de cations sulfonée sous la forme hydrogène.

7. Un procédé selon l'une quelconque des revendications précédentes, dans lequel la réaction d'éthérification est réalisée à une température dans la plage de 50 à 80°C.

8. Un procédé selon l'une quelconque des revendications précédentes, dans lequel la réaction d'éthérification est réalisée sous une pression dans la plage de 10 à 20 bars.

9. Un procédé selon l'une quelconque des revendications précédentes, dans lequel le rapport molaire entre l'alcool et l'oléfine tertiaire reactive est dans la plage de 0,3:1 à 1,5:1.

10. Un procédé selon l'une quelconque des revendications précédentes, dans lequel la vitesse spatiale horaire liquide de la charge d'alimentation est située dans la plage de 0,5 à 5 vol/vol/h.